# EUROPEAN PATENT SPECIFICATION

(11) **EP 4 100 089 B1**
(45) Date of publication and mention of the grant of the patent: **06.08.2025**
(21) Application number: 21750249.1
(22) Date of filing: 04.02.2021
(51) Int. Cl.: A61M 15/08

(54) **KIT FOR TREATING CHRONIC AND LONG-TERM NASAL CONGESTION**
KIT ZUR BEHANDLUNG VON CHRONISCHER UND LANGFRISTIGER VERSTOPFTER NASE
KIT DE TRAITEMENT DE CONGESTION NASALE CHRONIQUE ET À LONG TERME

(30) Priority: 06.02.2020 US 202062970794 P
(43) Date of publication of application: 14.12.2022
(73) Proprietor: Horizon IP Tech, LLC, Honolulu, HI 96813 (US)
(72) Inventor: IERULLI, Joseph V., Sarasota, Florida 34235 (US)
(74) Representative: Stanley, David William
(86) International application number: PCT/US2021/016536
(87) International publication number: WO 2021/158736

(56) References cited:
- US-A1- 2004 235 807
- US-A1- 2006 207 596
- US-A1- 2006 207 596
- US-A1- 2007 286 812
- US-A1- 2008 184 995
- US-A1- 2010 282 246
- US-A1- 2010 282 246
- US-A1- 2011 011 392
- US-A1- 2016 354 558
- US-A1- 2019 160 238
- US-B1- 6 516 795
- BISHOP COURTNEY A. ET AL: "Magnetic resonance imaging reveals the complementary effects of decongestant and Breathe Right Nasal Strips on internal nasal anatomy : Alleviation of Nasal Congestion Assessed with MRI", THE LARYNGOSCOPE, vol. 126, no. 10, 1 October 2016 (2016-10-01), United States, pages 2205 - 2211, XP093121908, ISSN: 0023-852X, DOI: 10.1002/lary.25906

## Description

This paragraph is blank.

### FIELD

The present invention relates to nasal congestion of the human nasal passages and is concerned particularly with nasal congestion treatment kits that comprise at least one first nasal-spray apparatus for administering a pharmaceutically-inactive solution as a spray mist into a nasal passage of a human nose; at least one second nasal-spray apparatus for administering a measured amount of a pharmaceutically-active solution as a spray mist into said nasal passage; and at least one nasal dilator. Nasal congestion is blockage of the nasal passages, also known as nasal blockage, nasal obstruction, blocked nose, stuffy nose, or stuffed up nose. Nasal congestion is usually due to swollen nasal membranes from inflammation of blood vessels.

Nasal congestion may be pathological (infection or rhinitis, for example) or natural. The latter refers to, for example, a deviated septum, or the Nasal Cycle (the result of alternating congestion and decongestion, controlled by the autonomic nervous system, of the nasal conchae or turbinates), or to the well-documented change in nasal airway resistance that occurs with posture change from sitting to supine (as in going to bed at night). The sensation of nasal obstruction upon lying down is a commonly related experience by patients in daily ENT care. The effect of posture change from the sitting to the supine position produces a decrease in nasal cross-sectional area and volume in both normal subjects and in subjects with symptoms of rhinitis, Roithman, R. et al (July/August 2005). Effects of posture change on nasal patency. Brazilian Journal of Otorhinolaryngology. 71 (4) Part 1. With the Nasal Cycle, the turbinates in one fossa fill up with blood while the opposite turbinates decongest by shunting blood away, the cycle having a mean duration of two and a half hours. The Nasal Cycle should not be confused with pathological nasal congestion. Individuals with normal nasal breathing sometimes become aware of the Nasal Cycle during sleep, particularly when sleeping on either side, and may be otherwise unaware of the Nasal Cycle unless there is an underlying nasal obstruction. Josephson, J. S. (2006). Sinus Relief Now: The Ground-Breaking 5-Step Program for Sinus, Allergy, And Asthma Sufferers. Penguin Group. p. 15*.* In pathological conditions, however, the Nasal Cycle may influence the symptoms thereof. Huizing, E. H.; de Groot, J. A. M. (2003). Functional Reconstructive Nasal Surgery. Thieme. p. 52*.*

### BACKGROUND

Potential treatments for nasal congestion include the following: breathing steam from the shower; humidifier or eucalyptus oil in boiling water; inhaling saline nasal spray; flushing the sinuses with a liquid nasal rinse; applying a warm compress; allergy medicine (antihistamine); decongestants (oral or spray); staying hydrated, *"*How to get rid of a stuffy nose: Ten possible treatments", Medical News Today, Healthline Media UK Ltd., 1 March 2017, http://www.medicalnewstoday.com/articles/313808.php*.* Treatments for chronic sinusitis include nasal corticosteroids, saline nasal irrigation, oral or injected corticosteroids, and aspirin desensitization, *"Chronic sinusitis", Mayo Clinic, Mayo Foundation for Medical Education and Research (MFMER), https:*//*www.mayoclinic.org*/*diseases-conditions*/*chronic-sinusitis*/*diagnosis-treatment*/*drc-20351667.* It should be noted that these treatments are listed singularly, with no suggestion of using them in combination.

The best singular treatment for episodic nasal congestion is cited as being an over-the-counter (OTC) medicated decongestant nasal spray, *"*How To Treat Nasal Congestion," WebMD, WebMD LLC, https://www.webmd.com/allergies/sinus-congestion#1*.* Non prescription decongestant nasal sprays comprise a direct acting sympathomimetic used as a vasoconstrictor to relieve nasal congestion, Martindale, The Extra Pharmacopoeia, 30th ed, p1251*.* The most common of these is Oxymetazoline (about 80% of all OTC decongestant nasal spray products), followed by Phenylephrine and Xylometazoline. Decongestant nasal sprays work by narrowing blood vessels in the lining of the nose. This reduces how much blood flows through the area so that swollen tissue inside the nose shrinks, which reduces airway resistance, allowing air to pass through more easily.

Saline nasal sprays and decongestant nasal sprays act on the posterior region of the nasal passages extending beyond the nasal valve area. The active ingredient in saline nasal spray is sodium chloride, which thins or breaks up mucous and irrigates the nasal passages, allowing them to drain more easily. Saline sprays typically include a moisturizing element to soothe the nasal passages. Saline sprays and decongestant nasal sprays share most inactive ingredients (typically comprising one or more compounds having bactericidal, fungicidal, antiseptic and preservative properties).

Nasal dilators for supporting, stabilizing, and dilating the nasal outer wall tissues overlying the nasal airway passages are well disclosed in the art, and are widely available in both internal and external varieties. Nasal dilators act on a portion of the anterior region of the nasal passages extending from near the nostril openings to the nasal valve area. Stabilized nasal outer walls and/or dilated nasal passages reduce nasal passage airflow resistance and may prevent collapse of the outer wall tissues during inhalation.

Internal nasal dilators may include an active type that is at least partially inserted into the nasal cavities and has a spring element that contacts the interior side of the nasal outer walls so as to urge them outward, and a passive type typically having a stent-like construction that is substantially or wholly inserted into the nasal cavity. Since internal nasal dilators are positioned within the nasal cavity, nasal airflow resistance may be correspondingly increased.

External nasal dilators (END) are generically referred to as *nasal strips* or *nasal dilator strips,* and are more widely used than internal nasal dilators. The END is flexed across the bridge of the human nose and adhered to the skin surfaces on each side thereof. The functional part of the END is at least one resilient member (synonymously referred to in the art as a *spring, spring member, resilient band, resilient member band, spring band,* or *bridge*) that extends along the length of the dilator. When flexed, the resilient member exerts a spring biasing force that urges the nasal passage outer wall tissues outward, stabilizing the outer walls and expanding, or dilating, the nasal passages. ENDs have been clinically found to improve nasal patency by increasing nasal passage cross-sectional area, nasal volume, and peak nasal inspiratory flow (PNIF). Improved nasal patency may have beneficial effects generally, may increase oxygen uptake, may improve sleep, may reduce sleep disturbances and nighttime awakenings, or may improve nasal snoring or obstructive sleep apnea (OSA).

ENDs and saline nasal sprays are both substantially benign and may be used frequently. Using an END and decongestant nasal spray together has been clinically shown to be cumulative or additive, Bishop et al, Magnetic Resonance Imaging Reveals the Complementary Effects of Decongestant and Breathe Right Nasal Strips on Internal Nasal Anatomy, The Laryngoscope, VC 2016 The American Laryngological, Rhinological and Otological Society, Inc.) *("*The Effect of External Nasal Dilators as Measure by Acoustic Rhinometry", B A Ng et al, ENT, Ear Nose & Throat Journal, October 1998*) ("*Nasal airflow dynamics: mechanisms and responses associated with an external nasal dilator strip"J.P. Kirkness et al, European respiratory Journal, Eur Respir J 2000; 15: 929±936*) (*Roithmann R, et al, "Role of the external nasal dilator in the management of nasal obstruction" Laryngoscope. 1998;108(5):712-715*.* However, overuse of decongestant nasal sprays is common. After three consecutive days a user may develop a well-documented condition known as *rebound congestion* or *rhinitis medicamentosa (RM).* RM means congestion (Rhinitis) caused by medication. RM occurs when the nasal passage turbinates are abnormally enlarged so as to block nasal airflow. Continued use of the nasal spray decongestant aggravates the problem and is counterproductive, resulting in a vicious cycle. Users may become physically and psychologically addicted, and may use several spray bottles weekly to keep the nasal passages open.

RM first appeared in medical literature in the 1930's. Prior to the mid-1960's, decongestant nasal sprays required a prescription. When these medications became available over the counter, incidents of rebound congestion increased. Online search queries related to RM are believed to total more than 2.4 million each year. Ear, Nose and Throat (ENT) clinics have reported that 5%-7% of their patients have RM. A worldwide RM patient population is estimated to be 7-10 million individuals.

The only known treatment for rebound congestion, or RM, is to stop using decongestant nasal spray, even though the nasal passages will become fully congested during withdrawal. It is believed that withdrawal efforts thus have a high failure rate, in that few individuals can tolerate the discomfort associated therewith. By halting decongestant nasal spray use, eventually the nasal passages will open again, typically over the course of days or weeks, Graf P. Rhinitis medicamentosa: a review of causes and treatment. Treat Respir Med. 2005; 4:21-29*.* Graf P. Rhinitis medicamentosa: aspects of pathophysiology and treatment. Allergy. 1997; 52(40 suppl):28-34*.* RameyJT, Bailen T, Lockey RF. Rhinitis medicamentosa. J Investig Allergol Clin Immunol. 2006; 16(3):148-155*.*

US patents 5988870 and 6712803 teach diluting decongestant nasal spray to address rebound congestion.

While decongestant nasal sprays have a 2-3 day 'safe period', episodic bouts of cough/cold/flu may last considerably longer. Additionally, chronic nasal congestion from other causes unrelated to infection or allergy may also last for considerably longer than the safe period for decongestant nasal spray use. There is thus an unmet need to provide apparatus and methods that: relieve nasal congestion with substantially the same efficacy as decongestant nasal sprays used as directed; may be used daily for longer than several consecutive days; and eliminate or reduce rebound congestion or a risk thereof.

US 2010/282246 Al (DJUPESLAND PER GISLE [NO] ET AL) 11 November 2010 (2010-11-11) discloses a nasal delivery device for and method of delivering substance to a nasal cavity of a subject.

Bishop Courtney A. ET AL: "Magnetic resonance imaging reveals the complementary effects of decongestant and Breathe Right Nasal Strips on internal nasal anatomy : Alleviation of Nasal Congestion Assessed with MRI" is published in The Laryngoscope, vol. 126, no. 10, 1 October 2016 (2016-10-01), pages 2205-2211, XP093121908, United States ISSN: 0023-852X, DOI: 10.1002/lary.25906

US 2004/235807 Al (WEINRICH KARL P [US] ET AL) 25 November 2004 (2004-11-25) discloses a pharmaceutical formulation provided for nasal drug administration of a topically administrable decongestant, a topically administrable corticosteroid and a pharmaceutically acceptable carrier.

### SUMMARY

According to the present invention, there is provided a nasal congestion treatment kit, comprising:
at least one first nasal-spray apparatus provided with a pharmaceutically-inactive solution and configured for administering said solution as a spray mist into a nasal passage of a human nose;
at least one second nasal-spray apparatus provided with a pharmaceutically-active solution and configured for administering a measured amount of said solution as a spray mist into said nasal passage; and
at least one nasal dilator: wherein:
   said pharmaceutically-active solution is an aqueous solution comprising not less than 0.002% and not more than 0.02% of a pharmaceutically-active ingredient by weight;
   said pharmaceutically-inactive solution is an aqueous solution comprising a salt selected from the group consisting of sodium chloride and potassium chloride; and
   said pharmaceutically active solution comprises a sympathomimetic vasoconstrictor as the pharmaceutically-active ingredient.

Preferably, each second nasal-spray apparatus is configured such that emission of an effective dose of said pharmaceutically-active solution as a spray mist requires at least one full actuation of the respective second nasal-spray apparatus, and each second nasal-spray apparatus is configured such that emission of an effective dose of said pharmaceutically-active solution as a spray mist requires not more than three full actuations of the respective second nasal-spray apparatus.

Preferably, said at least one nasal dilator consists of M nasal dilators, said at least one second nasal-spray apparatus contain, in total, N effective doses of said pharmaceutically-active solution, M is not more than 4 * N, and M is not less than N / 2.

Preferably, each first nasal-spray apparatus is configured such that a single full actuation of the respective first nasal-spray apparatus effects emission of a first amount of said pharmaceutically-inactive solution as a spray mist, and said first amount is at least five times said measured amount and less than twenty times said measured amount.

Preferably, an effective dose of said pharmaceutically-active solution comprises not less than 1 µg and not more than 10 µg of said pharmaceutically-active ingredient.

Preferably, said pharmaceutically-inactive solution comprises not less than 0.5% and not more than 0.8% of said salt per weight.

Preferred embodiments of the present invention may treat both episodic and chronic nasal congestion of the human nasal passages with the same efficacy as OTC decongestant vasoconstrictor nasal sprays but with a substantially reduced risk of incurring rebound congestion/RM. OTC decongestant nasal spray, saline nasal spray, and nasal dilators-though found individually in the prior art-are combined in embodiments of the present invention in new, novel, and unobvious ways: a substantial application of saline-based spray mist thins and loosens nasal mucous within the posterior region of the nasal passages; a small fractional application of the recommended therapeutic dose of a decongestant-based spray mist decongests the posterior region of the nasal passages; a nasal dilator increases nasal airflow by mechanically opening the nasal passages at the anterior region thereof; a minutes-long delaying period allows the complementary actions to slowly achieve a maximum effect.

An unexpected result was found by combining a small fraction of the recommended therapeutic dose of the vasoconstrictor nasal spray with a substantially greater volume of saline nasal spray. After delaying for roughly 5-20 minutes, a decongestant state appeared to be at least substantially the same as administering the recommended dose (2-3 actuations from a squeeze bottle or metered-dose pump, Oxymetazoline HCl 0.05% by weight/volume) of decongestant spray alone. Administration of the nasal sprays, the resting period, and the resultant decongestant effect may be enhanced by application of an END, particularly of an advanced type having a plurality of resilient spring branches extending outward from a common center. In one continuous period of use, the decongested state was repeatedly maintained throughout the nighttime sleep cycle for up to about sixty consecutive nights. Other periods of use averaged from about five to about ten consecutive nights. In all cases, nasal discharge did not increase by any significant amount, and the inventive method did not trigger rebound congestion, as described herein, at all.

A saline spray is provided in a first spray apparatus and a vasoconstrictor decongestant spray in a second spray apparatus, so as to allow the user to administer an unrestricted amount of saline spray and a pre-determined limited amount of vasoconstrictor nasal spray. The present invention also provides nasal dilators, first and second spray apparatus co-packaged in kit form whereby a user may practice the inventive method.

In describing nasal sprays, related apparatus, and the use thereof, the terms *"delivered"* and *"emitted"* herein refer to an amount or volume for which a spray apparatus is configured to deliver or emit upon an actuation. The term *"administered"* refers herein to a total amount or volume resulting from one or more intended user actuations of a spray apparatus.

It is believed that embodiments of the present invention may provide substantially the same efficacy as OTC vasoconstrictor decongestant nasal sprays used alone as recommended on the product package labeling, but embodiments of the invention may be used daily for significantly longer than the 2-3 day decongestant nasal spray safe period, wherein a risk of rebound congestion is at least substantially reduced. Embodiments of the present invention may have the greatest potential benefit for naturally-caused chronic nasal congestion, as described herein, particularly where impacted by pathological causes.

### BRIEF DESCRIPTION OF DRAWINGS

Fig. 1 is a side view of a human nose shown as a portion of a human face with a cross-sectional representation by stippling of the nasal passages thereof.
Fig. 2 is a perspective view of the human nose depicted in Figs. 1 and 3, showing by stippling a cross-sectional representation of the nasal valve area.
Fig. 3 is an underside elevational view of the human nose depicted in Fig. 1.
Fig. 4 is a perspective view of a human nose showing an END applied thereon.
Fig. 5 is a plan view of labeling for oxymetazoline-based OTC decongestant nasal spray.
Fig. 6 illustrates articles as may be found in a kit, in accordance with the present invention, for use in treating long-term or chronic nasal congestion.
Fig. 7 is a perspective view of a human nose showing an END applied thereon.

### DETAILED DESCRIPTION

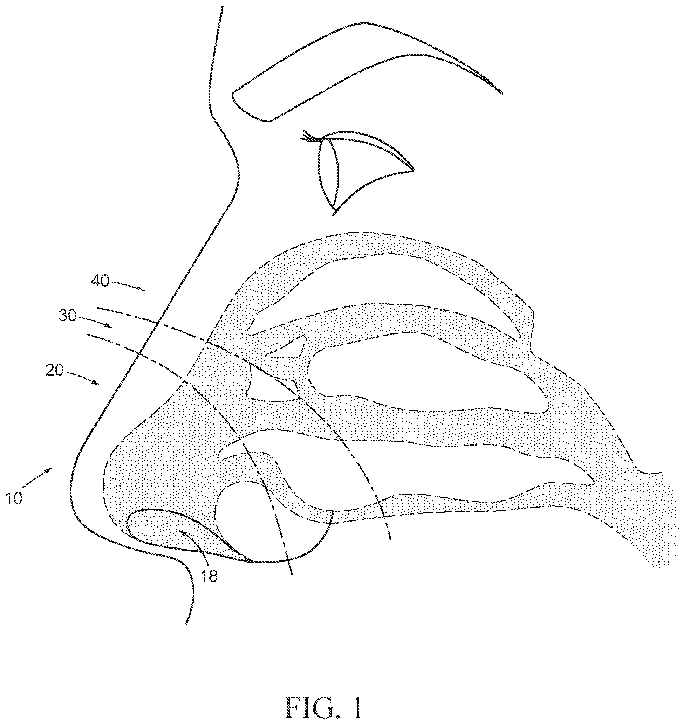

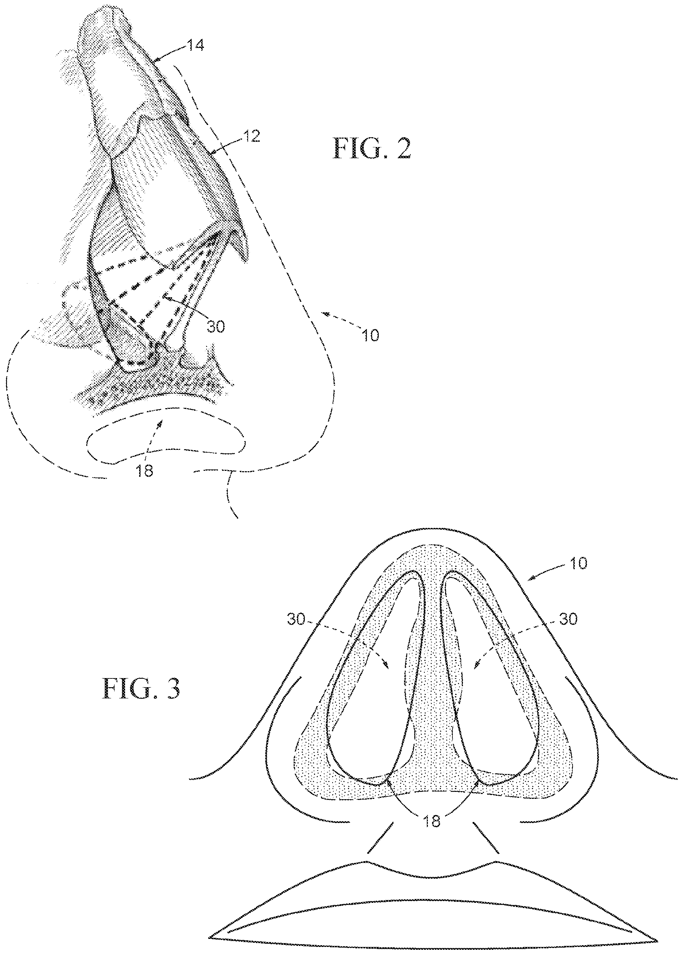

Several views of a human nose, **10,** are shown in Figs. 1-3. Stippling bounded by dashed lines in Fig. 1 depicts an approximate cross-sectional rendering of the nasal passages extending from the nostril opening, **18,** to the back of the throat. The nasal passages of nose **10** comprise an anterior region, also known as the nasal vestibule, **20,** extending from nostril opening (or external nasal valve), **18,** to the internal nasal valve area, **30**, and a posterior region, **40,** extending beyond nasal valve area **30.** Arcuate broken lines delineate approximate boundaries between the anterior and posterior regions and the nasal valve area. Dark dashed lines in Fig. 2 more specifically depict nasal valve area **30** as an inverted cone-shape (illustrated on only one side of the nasal septum). For additional perspective, Fig. 2 also illustrates the upper lateral cartilage, **12,** and the nasal bone, **14.** Fig. 3 also shows, by stippling bounded by dashed lines, a cross-section of nasal valve area **30** from a direction perpendicular to that shown in Fig. 1. It is noted that the nasal passages are narrower at nasal valve area **30** compared to at nostril opening **18.**

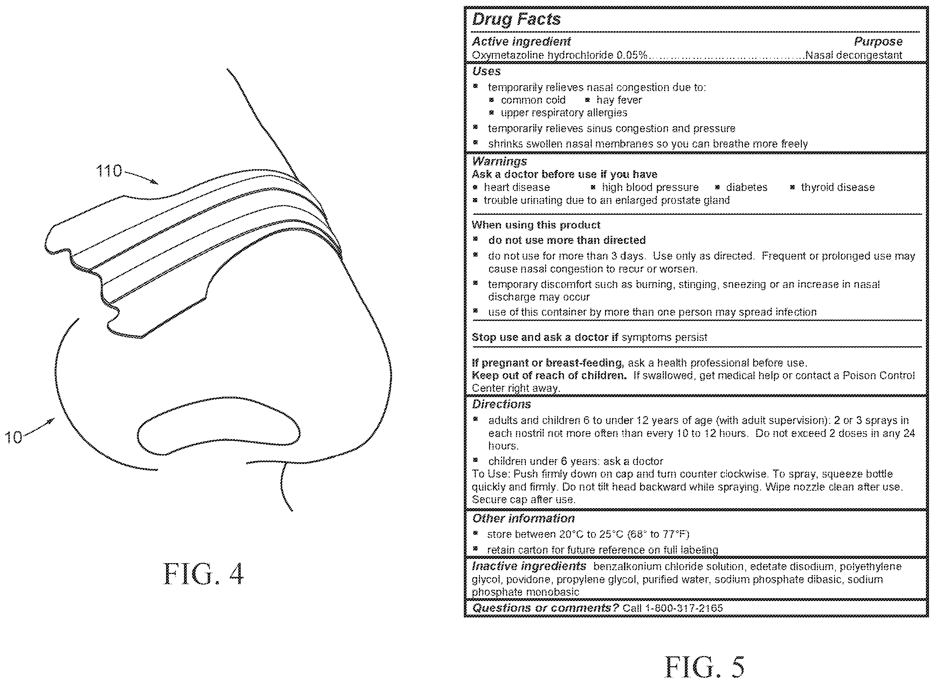

Fig. 4 shows a generic END, **110,** in use on nose **10.** An END is most preferred over an internal nasal dilator of any type (not shown), since the very presence of an internal nasal dilator within the nasal passages increases airway resistance thereat. The general construction and range of spring biasing force provided by END **110** is preferably consistent with widely commercially available nasal dilators. However, as described hereinbefore, END **110** primarily acts on nasal valve area **30** of the nasal passages, and, depending on how it is constructed, may act on substantial portions of anterior region **20** thereof, as well as portions extending slightly into posterior region **40** thereof. The END art is continually advancing; exemplary of an END that acts on portions of the nasal passages above and below the nasal valve is disclosed, for example, in U.S. Patent No. 10,893,971. It is believed that an END, **120,** of this type has a greater beneficial effect on the present method than does generic END **110,** and a far greater effect than an internal nasal dilator. Applying a nasal dilator may be performed at any point in the sequence of steps, but is most preferably applied prior to administering a first nasal spray, as described hereinbelow. As noted herein, inhaled nasal sprays act primarily on posterior region **40.**

Instructions for administering nasal spray via a squeeze bottle or pump actuator on oneself, or for administration to patients by a health professional, are published online, for example, by the University of Illinois-Chicago Drug Information Group (*https:*//*www.healthline.com*/*health*/*general-use*/*how-to-use-nasal-spray.* An END is applied by centering it horizontally along the centerline of the bridge of the nose, and vertically substantially over nasal valve area **30.** Depending on its width, the END may extend partially over nasal vestibule **20** adjacent the nasal valve.

The step of applying an END notwithstanding, a user actuates a first spray apparatus and inhales a first spray mist of a first aqueous solution into at least one nostril opening of the nose. The first aqueous solution comprises sodium chloride and water in a saline solution, as may be found in the art, suitable for inhaling into the nasal passages, and which acts to thin and loosen mucous deposits within posterior region **40** thereof. The total mist volume administered, whether from one actuation or a plurality of actuations, is unrestricted, but is preferably at least significantly greater than the prescribed volume administered from the second aqueous solution. This may vary substantially from user to user, and even from use to use. However, a suitable starting ratio between the first and second spray mists, for example, may be roughly an order of magnitude. That is, a total mist plume volume of about 100 microliters (µl) from the first spray mist (whether by a single emitted plume or plurality thereof) may correspond to roughly 10 µl from the second spray mist. The administered volume of the first aqueous solution may be otherwise user-determined.

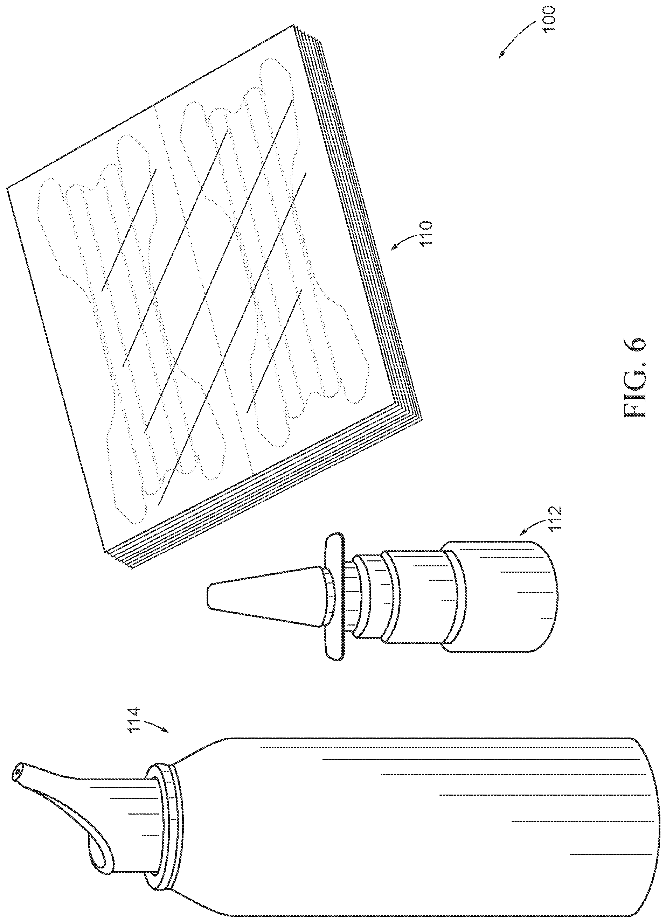

The first spray apparatus may comprise a plastic squeeze bottle (not shown), a metered dose spray pump, **112,** or a pressurized metal spray canister, **114,** as seen, for example, in Fig. 6. The spray mist volume from a squeeze bottle may vary depending on how firmly it is squeezed, and may thus require some repetition in order to deliver a suitable or desired amount of saline mist. However, spray canister **114** delivers a continuous mist plume or liquid spray for as long as the actuator is depressed, and is thus most preferred. For example, one actuation may be sustained for the duration of one long, sharp, nasal inhalation, resulting in a substantial and significant emitted volume of spray or mist, which may more easily correspond to the aforementioned order of magnitude.

The user next actuates a second spray apparatus and inhales a second spray mist of a second aqueous solution into the at least one nostril opening. The second aqueous solution includes therein a pharmaceutically-active ingredient in the form of a sympathomimetic vasoconstrictor, most preferably oxymetazoline HCl. Inactive ingredients of the second aqueous solution are consistent with that widely found in the prior art.

The second spray apparatus most preferably comprises a spray pump, as may be provided, for example, by Aptargroup (Congers, NY). The second aqueous solution is most preferably regulated or restricted by configuration of the spray apparatus and/or by the amount or proportion of the pharmaceutically-active ingredient relative, by weight, to the volume of aqueous solution. For example, the second spray apparatus mechanism (pump or orifice) may be configured to deliver a smaller mist plume volume than typically found in OTC nasal decongestants, and thus deliver a fraction of the therapeutically recommended dose thereof. Additionally, or alternatively, the second aqueous solution may contain a lesser concentration of the pharmaceutically-active ingredient than that typically found in OTC nasal decongestants, and thus deliver a fraction of the recommended dose thereof.

Widely available OTC nasal decongestant sprays in a squeeze bottle or spray pump typically comprise oxymetazoline HCl 0.05% by weight/volume. A single full upright actuation (squeeze or plunge) is believed to deliver a 28.9 ± 6.8 µl mist plume (28,900 ± 6,800 micrograms), thus including therein about 14.45 ± 3.4 micrograms (mcg) of oxymetazoline (0.05% by weight thereof). As seen in Fig. 5, the therapeutically recommended dose for OTC oxymetazoline-based decongestant nasal sprays is 2-3 such actuations, for a total of about 29 to 43 mcg of oxymetazoline.

The smallest effective fractional dose of pharmaceutically-active ingredient, in accordance with the present invention, is in a range that may vary from user to user and use to use. A preferred range of pharmaceutically-active ingredient is estimated to be between about 1 mcg and roughly 10 mcg, a more preferred range may be from about 2 mcg to about 8 mcg, and a still more preferred range may be from about 3 mcg to about 6 mcg. Again, the unexpected result of the present invention is that so little pharmaceutically-active ingredient may be effective when combined with a comparably voluminous application of saline spray.

In any case, the user is cautioned to use no more than a predetermined number of sprays corresponding to the aforementioned preferred range. For example, where the second spray apparatus is configured to emit a 30µl (30,000 mcg) plume per actuation, including therein the pharmaceutically-active ingredient at 0.005% by weight, the user would administer 1.5 mcg of the vasoconstrictor per actuation. According to an embodiment the user may thus conveniently administer a suitable dose with one, two or three spray actuations (1.5, 3.0, or 4.5 mcg, respectively). Beneficially, this is similar to the number of actuations that the user may already be accustomed to in using widely available OTC nasal decongestant sprays. Thus, an embodiment may comprise a standard sympathomimetic vasoconstrictor in its standard packaging, but at a substantially diluted concentration (0.005% by weight), well below the concentrations typically expected to be therapeutically effective (0.05% by weight).

It will be apparent to one or ordinary skill in the art that the second spray apparatus and the second aqueous solution may have a variety of configurations/concentrations relative to each other while remaining within the preferred range of the pharmaceutically-active ingredient administered.

Alternatively, the second spray apparatus may be selected from typical widely available OTC nasal decongestant sprays having a squeeze bottle or spray pump apparatus comprising oxymetazoline HCl 0.05%. However, for purposes of the present method, the user must carefully actuate the apparatus less firmly so as to deliver a lesser volume of spray mist, and thus a lesser amount of oxymetazoline from a single actuation, than that for which the spray apparatus is configured. A partial single plunge may thus deliver a mist plume in a range, for example, of from about 5 µl (5,000 micrograms) to about 20 µl (20,000 micrograms), including therein from about 2.5 mcg to about 10 mcg of oxymetazoline (0.05% thereof). Though less precise, this alternative step has been found to be workable.

After administering nasal spray-by first and second sprays-the user delays for a period of from about five minutes to about twenty minutes, preferably in the supine position, to allow effect of a recommended dose of a sympathomimetic vasoconstrictor decongestant nasal spray used alone, further repetition thus being unnecessary.

The user may repeat the inventive method, most preferably no more than once daily, but permissibly for more than three consecutive days or nights, wherein a risk of rebound congestion or rhinitis medicamentosa is at least substantially reduced.

An example of situational implementation of the inventive method and results therefrom may be as follows: A user applies an END at bedtime. Asleep on one side, the user is later awakened by substantial congestion of the nasal passage on the 'low' side, perhaps the result of the natural Nasal Cycle, perhaps exacerbated by mild pathological cause. The user sits upright, administers first and second nasal sprays into the congested nasal passage, as described herein, and lays back down in the supine position. Some partial decongestion occurs within several minutes, more pronounced decongestion some minutes thereafter accompanied by an urge to inhale sharply with post nasal drainage and substantial nasal clearing. Returning to the one side, the user senses substantial airflow improvement and returns to sleep. The treated nasal passage remains in a decongested state for the remainder of the sleep cycle, regardless of which side the user lays on.

As further seen in Fig. 6, a kit, **100,** for treating nasal congestion using the present method comprises aqueous solution in first and second spray apparatus 112, 114, as described hereinbefore, and at least one nasal dilator. An END as described herein is preferred; a plurality thereof may, for example, correspond approximately to the number of metered doses of the second nasal spray apparatus. The kit may further include instructions for administering first and second nasal spray mists, as described herein, and instructions for applying a nasal dilator.

A nasal-congestion treatment kit according to this embodiment may be co-packaged into a fitted container, not shown, where each component is arranged in order of use according to an embodiment. The instruction guide may be color-coded or otherwise presented to facilitate the user's correct execution of the method. Further, refill components may be provided to replace any individual component that has been exhausted through previous uses. The extra spray apparatus (saline spray, pharmaceutically-active spray, or combination spray apparatus) and the plurality of nasal dilators may be packaged to conform to the kit container.

The applications of the present invention have been described largely by reference to specific examples and in terms of particular allocations of functionality to certain physical components or collections of components. However, those of skill in the art will recognize that nasal congestion can also be treated effectively with a reduced risk of causing rebound congestion or rhinitis medicamentosa by apparatus and kits that distribute the functions of embodiments of this invention differently than herein described. Such variations and implementations are understood to be captured according to the following claims.

## Claims

1. A nasal congestion treatment kit (100), comprising:
at least one first nasal-spray apparatus (112,114) provided with a pharmaceutically-inactive solution and configured for administering said solution as a spray mist into a nasal passage of a human nose;
at least one second nasal-spray apparatus (112,114) provided with a pharmaceutically-active solution and configured for administering a measured amount of said solution as a spray mist into said nasal passage; and
at least one nasal dilator (110);
wherein:
said pharmaceutically-active solution is an aqueous solution comprising not less than 0.002% and not more than 0.02% of a pharmaceutically-active ingredient by weight;
said pharmaceutically-inactive solution is an aqueous solution comprising a salt selected from the group consisting of sodium chloride and potassium chloride; and
said pharmaceutically active solution comprises a sympathomimetic vasoconstrictor as the pharmaceutically-active ingredient.

2. The nasal congestion treatment kit (100) of claim 1, wherein:
each second nasal-spray apparatus (112,114) is configured such that emission of an effective dose of said pharmaceutically-active solution as a spray mist requires at least one full actuation of the respective second nasal-spray apparatus (112,114), and
each second nasal-spray apparatus (112,114) is configured such that emission of an effective dose of said pharmaceutically-active solution as a spray mist requires not more than three full actuations of the respective second nasal-spray apparatus (112,114).

3. The nasal congestion treatment kit (100) of claim 1 or 2, wherein:
said at least one nasal dilator (110) consists of M nasal dilators,
said at least one second nasal-spray apparatus (112,114) contain, in total, N effective doses of said pharmaceutically-active solution, M is not more than 4 * N, and M is not less than N / 2.

4. The nasal congestion treatment kit (100) of any one of the preceding claims, wherein:
each first nasal-spray apparatus (112,114) is configured such that a single full actuation of the respective first nasal-spray apparatus (112,114) effects emission of a first amount of said pharmaceutically-inactive solution as a spray mist, and
said first amount is at least five times said measured amount and less than twenty times said measured amount.

5. The nasal congestion treatment kit (100) of any one of the preceding claims, wherein:
an effective dose of said pharmaceutically-active solution comprises not less than 1 µg and not more than 10 µg of said pharmaceutically-active ingredient.

6. The nasal congestion treatment kit (100) of any one of the preceding claims, wherein:
said pharmaceutically-inactive solution comprises not less than 0.5% and not more than 0.8% of said salt per weight.

## Patentansprüche

1. Kit (100) zur Behandlung von verstopfter Nase, umfassend:
mindestens eine erste Nasensprühvorrichtung (112,114), die mit einer pharmazeutisch inaktiven Lösung versehen ist und konfiguriert ist zum Verabreichen der Lösung als Sprühnebel in einen Nasengang einer menschlichen Nase;
mindestens eine zweite Nasensprühvorrichtung (112,114), die mit einer pharmazeutisch aktiven Lösung versehen ist und konfiguriert ist zum Verabreichen einer abgemessenen Menge der Lösung als Sprühnebel in den Nasengang; und
mindestens einen Nasendilatator (110);
wobei:
die pharmazeutisch aktive Lösung eine wässrige Lösung ist, die nicht weniger als 0,002 % und nicht mehr als 0,02 % eines pharmazeutisch aktiven Inhaltsstoffs nach Gewicht umfasst;
die pharmazeutisch inaktive Lösung eine wässrige Lösung ist, die ein Salz umfasst, das ausgewählt ist aus der Gruppe bestehend aus Natriumchlorid und Kaliumchlorid;
und
die pharmazeutisch aktive Lösung einen sympathomimetischen Vasokonstriktor als pharmazeutisch aktiven Inhaltsstoff umfasst.

2. Kit (100) zur Behandlung von verstopfter Nase nach Anspruch 1, wobei:
jede zweite Nasensprühvorrichtung (112,114) so konfiguriert ist, dass die Emission einer wirksamen Dosis der pharmazeutisch aktiven Lösung als Sprühnebel mindestens eine vollständige Betätigung der jeweiligen zweiten Nasensprühvorrichtung (112,114) erfordert, und
jede zweite Nasensprühvorrichtung (112,114) so konfiguriert ist, dass die Emission einer wirksamen Dosis der pharmazeutisch aktiven Lösung als Sprühnebel nicht mehr als drei vollständige Betätigungen der jeweiligen zweiten Nasensprühvorrichtung (112,114) erfordert.

3. Kit (100) zur Behandlung von verstopfter Nase nach Anspruch 1 oder 2, wobei:
der mindestens eine Nasendilatator (110) aus M Nasendilatatoren besteht,
die mindestens eine zweite Nasensprühvorrichtung (112,114) insgesamt N wirksame Dosen der pharmazeutisch aktiven Lösung enthält, M nicht mehr als 4 * N ist und M nicht weniger als N / 2 ist.

4. Kit (100) zur Behandlung von verstopfter Nase nach einem der vorhergehenden Ansprüche, wobei:
jede erste Nasensprühvorrichtung (112,114) so konfiguriert ist, dass eine einzige vollständige Betätigung der jeweiligen ersten Nasensprühvorrichtung (112,114) die Emission einer ersten Menge der pharmazeutisch inaktiven Lösung als Sprühnebel bewirkt, und
die erste Menge mindestens das Fünffache der abgemessenen Menge und weniger als das Zwanzigfache der abgemessenen Menge beträgt.

5. Kit (100) zur Behandlung von verstopfter Nase nach einem der vorhergehenden Ansprüche, wobei:
eine wirksame Dosis der pharmazeutisch aktiven Lösung nicht weniger als 1 µg und nicht mehr als 10 µg des pharmazeutisch aktiven Inhaltsstoffs umfasst.

6. Kit (100) zur Behandlung von verstopfter Nase nach einem der vorhergehenden Ansprüche, wobei:
die pharmazeutisch inaktive Lösung nicht weniger als 0,5 % und nicht mehr als 0,8 % des Salzes nach Gewicht umfasst.

## Revendications

1. Kit de traitement de congestion nasale (100), comprenant :
au moins un premier appareil de pulvérisation nasale (112,114) doté d'une solution pharmaceutiquement inactive et conçue pour administrer ladite solution sous la forme d'un brouillard de pulvérisation dans une voie nasale d'un nez humain ;
au moins un second appareil de pulvérisation nasale (112,114) doté d'une solution pharmaceutiquement active et conçue pour administrer une quantité mesurée de ladite solution sous la forme d'un brouillard de pulvérisation dans ladite voie nasale ; et
au moins un dilatateur nasal (110) :
ladite solution pharmaceutiquement active étant une solution aqueuse ne comprenant pas moins de 0,002 % et pas plus de 0,02 % d'un ingrédient pharmaceutiquement actif en poids ;
ladite solution pharmaceutiquement inactive étant une solution aqueuse comprenant un sel choisi dans le groupe constitué par le chlorure de sodium et le chlorure de potassium ; et
ladite solution pharmaceutiquement active comprenant un vasoconstricteur sympathomimétique en tant qu'ingrédient pharmaceutiquement actif.

2. Kit de traitement de congestion nasale (100) selon la revendication 1 :
chaque second appareil de pulvérisation nasale (112,114) étant conçu de sorte que l'émission d'une dose efficace de ladite solution pharmaceutiquement active sous la forme d'un brouillard de pulvérisation nécessite au moins un actionnement complet du second appareil de pulvérisation nasale respectif (112,114), et
chaque second appareil de pulvérisation nasale (112,114) étant conçu de sorte que l'émission d'une dose efficace de ladite solution pharmaceutiquement active sous la forme d'un brouillard de pulvérisation ne nécessite pas plus de trois actionnements complets du second appareil de pulvérisation nasale respectif (112,114).

3. Kit de traitement de congestion nasale (100) selon la revendication 1 ou 2 :
ledit au moins un dilatateur nasal (110) se composant de M dilatateurs nasaux,
ledit au moins un second appareil de pulvérisation nasale (112,114) contenant, au total, N doses efficaces de ladite solution pharmaceutiquement active, M n'étant pas supérieur à 4 * N, et M n'étant pas inférieur à N / 2.

4. Kit de traitement de congestion nasale (100) selon l'une quelconque des revendications précédentes :
chaque premier appareil de pulvérisation nasale (112,114) étant conçu de sorte qu'un seul actionnement complet du premier appareil de pulvérisation nasale respectif (112,114) entraîne l'émission d'une première quantité de ladite solution pharmaceutiquement inactive sous la forme d'un brouillard de pulvérisation, et
ladite première quantité représentant au moins cinq fois ladite quantité mesurée et moins de vingt fois ladite quantité mesurée.

5. Kit de traitement de congestion nasale (100) selon l'une quelconque des revendications précédentes :
une dose efficace de ladite solution pharmaceutiquement active ne comprenant pas moins de 1 µg et pas plus de 10 µg dudit ingrédient pharmaceutiquement actif.

6. Kit de traitement de congestion nasale (100) selon l'une quelconque des revendications précédentes :
ladite solution pharmaceutiquement inactive ne comprenant pas moins de 0,5 % et pas plus de 0,8 % dudit sel en poids.
